# DEMANDE DE BREVET EUROPEEN

(11) **EP 0 712 630 A2**
(43) Date de publication de la demande: **22.05.1996**
(21) Numéro de dépôt: 95402550.8
(22) Date de dépôt: 15.11.1995
(51) Int. Cl.: A61K 33/04

(54) **Composition orale pour la prévention des allergies solaires à base d'un caroténoide, d'un tocophérol, d'acide ascorbique et de sélénium**

(30) Priorité: 17.11.1994 FR 9413783
(71) Demandeur: JCB COSMETIQUES, F-92300 Levallois Perret (FR)
(72) Inventeur: Blime, Jean-Claude, F-75017 Paris (FR)
(74) Mandataire: Burtin, Jean-François

(57) **Abrégé**

La présente invention concerne une formulation orale sous forme de capsule à ingérer, contenant dans un milieu physiologiquement acceptable, au moins :
(A) un caroténoïde
(B) un tocophérol
(C) l'acide ascorbique et
(D) du sélénium,

destinée au traitement des allergies solaires du type lucite estivale bénigne.

## Description

La présente invention a pour objet une composition orale pour la prévention des allergies solaires telles que les lucites estivales bénignes.

La lucite est un état inflammatoire provoqué sur la peau par l'exposition à la lumière et particulièrement au soleil, aux ultra-violets. La majorité des lucites observées sont des lucites estivales bénignes. Elles sont caractérisées par une éruption survenant en général au deuxième ou troisième jour d'exposition solaire. Cette photodermatose touche essentiellement les femmes ; elle est déclenchée principalement par les rayons UV-A.

On sait que les rayons UV-A provoquent le brunissement de la peau. Le phénomène de bronzage résulte de l'action de ces rayonnements sur les mélanocytes qui sont les cellules spécifiques de la périphérie du derme, responsables de la mélanogénèse. Un bon fonctionnement de cette réaction devrait avoir pour conséquence directe de produire un filtre naturel, le bronzage, qui assurerait une protection permanente.

Etant donné que cette photodermatose est déclenchée par les rayons UV-A de longueur d'onde 320 à 400 nm, les écrans solaires classiques qui ont un haut pouvoir filtrant en UV-B et un faible pouvoir filtrant en UV-A, sont généralement inefficaces dans la prévention des lucites estivales et peuvent même entraîner une aggravation de cette affection cutanée.

La demanderesse a découvert de manière surprenante que la protection des cellules aux rayonnements UV-A pouvait être renforcée par l'absorption, par voie orale, d' une formulation bronzante associant un caroténoïde, un tocophérol l'acide ascorbique (vitamine C) et du sélénium, et que cette formulation orale permettait de traiter efficacement, de manière préventive, les allergies solaires du type lucite estivale bénigne. De plus, cette association optimise la réaction de pigmentation de la peau au soleil

La protection des cellules contre les radiations UV est ainsi réalisée par ce complexe auto-régénérant fonctionnant de manière graduelle : la vitamine C protège le tocophérol qui est lui-même protégé par le caroténoïde ; le sélénium protège la vitamine C en stimulant l'activité de la Glutathion-Peroxidase et favorise la mélanogénèse noire, filtre naturel, préférentiellement à la mélanogénèse rouge. La pigmentation ainsi obtenue permet d'acquérir une protection contre les agressions responsables des lucites.

Les caroténoïdes utilisés selon la présente invention sont choisis de préférence parmi le β-carotène, l'α-carotène et les lycopènes. Ils sont utilisés dans la composition de l'invention à des doses allant de 60 à 150 mg par jour, soit de 15 à 37,5 mg par unité par prise. Ils peuvent aussi être utilisés sous forme d'extrait de carotte riche en β-carotène.

Les tocophérols utilisés selon l'invention sont choisis notamment parmi l'α-tocophérol ou vitamine E, le β-tocophérol, le γ-tocophérol, le δ-tocophérol, l'ε-tocophérol, le ξ₁-tocophérol, le ξ₂-tocophérol, le η-tocophérol ou les esters de tocophérol comme le succinate d'α-tocophérol. Ils sont utilisés dans les compositions de l'invention à des doses allant de 40 à 120 mg par jour, soit de 10 à 30 mg par unité de prise.

Le sélénium est utilisé à des doses minimales de 25 µg par jour, soit 6,25 µg par unité de prise.

L'acide ascorbique ou vitamine C est utilisé à des doses minimales de 120 mg par jour dans les compositions de l'invention, soit 30 mg environ par prise unitaire.

Le milieu physiologiquement acceptable qui constitue l'excipient des formulations de l'invention, comprend de préférence une huile d'origine végétale telle que l'huile de germe de ble, l'huile d'onagre, l'huile de soja, l'huile de bourrache ou l'huile de palme ; et/ou de la lécithine de soja et/ou de l'amidon et/ou du sucre. Il contient éventuellement un agent de sapidité comme un extrait naturel de fruits.

Les compositions selon l'invention peuvent contenir des agents d'enrobage tels que la gélatine, le sucre ou la glycérine.

Les compositions orales selon l'invention sont solides ou liquides de préférence elles sont conditionnées sous forme de capsules à ingérer, administrées quatre fois par jour, quize jours avant l'exposition solaire et pendant toute la durée du séjour où le sujet est exposé au soleil.

Les exemples qui suivent servent à illustrer l'invention sans toutefois présenter un caractère limitatif.

### EXEMPLES DE FORMULATION

### EXEMPLE 1 - 4 capsules par jour

| | |
|---|---|
| Beta-carotène | 15 mg |
| Vitamine C | 30 mg |
| α-tocophérol | 10 mg |
| Sélénium | 6,25 µg |
| Huile de soja | 190 mg |

### EXEMPLE 2 - 4 capsules par jour

| | |
|---|---|
| Alpha-carotène | 20 mg |
| Vitamine C | 30 mg |
| δ-tocophérol | 12 mg |
| Lycopène | 2 mg |
| Sélénium | 8 mg |
| Huile d'onagre | 120 mg |

### EXEMPLE 3 - 4 capsules par jour

| | |
|---|---|
| Extrait de carotte | 30 mg |
| Vitamine C | 30 mg |
| ε-tocophérol | 12 mg |
| Sélénium | 8 mg |
| Huile de soja hydrogénée | 180 mg |

### EFFICACITE THERAPEUTIQUE D'UNE FORMULATION SELON L'INVENTION SUR LES LUCITES ESTIVALES

On utilise la composition de l'exemple 1, sous la forme de capsules à ingérer sur 35 patients à raison de 4 capsules par jour (60 mg de β-carotène) 15 jours avant l'exposition solaire, puis pendant toute la durée de l'exposition du patient au soleil durant un séjour.

A la fin du séjour, chaque patient est interrogé sur les effets thérapeutiques préventifs de la formulation.

Elle est considérée comme :
Excellente : si aucune éruption cutanée n'est survenue
Bonne : s'il y a apparition de symptômes mineurs
Médiocre : s'il y a apparition de lésions cutanées
Nulle : s'il y a apparition de lucites estivales.

Les résultats obtenus sont indiqués dans le tableau suivant :

| APPRECIATION | EXCELLENTE | BONNE | MEDIOCRE | NULLE |
|---|---|---|---|---|
| NOMBRE DE PATIENTS | 12 | 17 | 5 | 1 |
| POURCENTAGE DE PATIENTS | 34,3 % | 48,6% | 14,3% | 2,8% |

Il en résulte que 82,9 % des sujets auxquels la formulation de l'invention a été administrée ont considéré que celle-ci était efficace pour la prévention des lucites estivales bénignes.

## Revendications

1. Composition orale médicamenteuse contenant dans un milieu physiologiquement acceptable, au moins :
(A) un caroténoïde ;
(B) un tocophérol ;
(C) l'acide ascorbique ; et
(D) du sélénium,
en association ou en mélange avec un excipient ou un véhicule approprié.

2. Utilisation d'une composition orale, selon la revendication 1, contenant dans un milieu physiologiquement acceptable, au moins :
(A) un caroténoïde ;
(B) un tocophérol ;
(C) l'acide ascorbique ; et
(D) du sélénium,
pour la préparation d'un médicament destiné au traitement des allergies solaires du type lucite estivale bénigne.

3. Utilisation, selon la revendication 2, caractérisée par le fait que le caroténoïde est présent à des doses de 60 à 150 mg/jour.

4. Utilisation, selon l'une quelconque des revendications 2 à 3, caractérisée par le fait que le tocophérol est présent à des doses de 40 à 120 mg/jour.

5. Utilisation, selon l'une quelconque des revendications 2 à 4, caractérisée par le fait que le sélénium est présent à des doses minimales de 25µg/jour.

6. Utilisation, selon l'une quelconque des revendications 2 à 5, caractérisée par le fait que l'acide ascorbique est présent à des doses minimales de 120 mg/jour.

7. Utilisation, selon l'une quelconque des revendications 2 à 6, caractérisée par le fait que le caroténoïde est choisi parmi le β-carotène, l'α-carotène et les lycopènes

8. Utilisation, selon l'une quelconque des revendications 2 à 7, caractérisée par le fait que le tocophérol est choisi parmi l'α-tocophérol ou vitamine E, le β-tocophérol, le γ-tocophérol, le δ-tocophérol, , l'ε-tocophérol, le ξ₁-tocophérol, le ξ₂-tocophérol, le η-tocophérol et le succinate d'α-tocophérol.

9. Utilisation, selon l'une quelconque des revendications 2 à 8, caractérisée par le fait que le milieu physiologiquement acceptable de la composition orale est constitué d'une huile végétale, de lécithine de soja, d'amidon, de sucre ou de leurs mélanges, et éventuellement d'un extrait naturel de fruits.

10. Utilisation, selon l'une quelconque des revendications 2 à 9, caractérisée par le fait que la composition orale contient en plus un agent d'enrobage.

11. Utilisation, selon l'une quelconque des revendications 2 à 10, caractérisée par le fait que la composition orale est conditionnée sous forme de capsule à ingérer.

12. Utilisation, selon l'une quelconque des revendications 2 à 11, caractérisée par le fait que la composition orale est administrée quatre fois par jour.
